# EUROPÄISCHE PATENTANMELDUNG

(11) **EP 1 174 181 A1**
(43) Veröffentlichungstag der Anmeldung: **23.01.2002**
(21) Anmeldenummer: 01114084.5
(22) Anmeldetag: 09.06.2001
(51) Int. Cl.: B01J 13/04, B01J 13/22

(54) **Mikrokapsel, Verfahren zu ihrer Herstellung, Verwendung derselben und Beschichtungsflüssigkeit mit solcher**

(30) Priorität: 18.07.2000 DE 10035302
(71) Anmelder: Deotexis Inc., New York, N.Y. 10022-4838 (US)
(72) Erfinder: Tebbe, Gerold, 98000 Monte Carlo (MC)
(74) Vertreter: Ostertag, Reinhard

(57) **Zusammenfassung**

Thermostatisierende Mikrokapseln (10) haben einen Kern (12), der aus einem Material besteht, das einen Schmelzpunkt im Bereich zwischen 5°C und 40°C aufweist. Der Kern ist von einer ersten Wand (14) umgeben, die durch Umspritzen eines Kerntropfens erhalten ist. Die Wand (14) kann unvollständig sein, so daß Kernmaterial noch in sie eingelagert ist. Die erste Wand (14) ist von einer weiteren Wand (16) umgeben, die in einem getrennten Verfahrensschritt, nämlich in einem Wirbelbettverfahren erzeugt wurde. In der so erhaltenen Mikrokapsel (10) ist das Kernmaterial (12) sicher und dicht eingeschlossen.

## Beschreibung

Die Erfindung betrifft eine Mikrokapsel gemäß dem Oberbegriff von Anspruch 1, ein Verfahren zu ihrer Herstellung, ihre Verwendung und eine zum Auftragen solcher Mikrokapseln geeignete Beschichtungsflüssigkeit.

Es ist bekannt, daß Materialien im Hohlraum (Kernmaterialien) von Mikrokapseln durch Schmelzen und Erstarren für thermostatisierende Zwecke eingesetzt werden können, da sie beim Schmelzen der Umgebung Wärme entziehen und beim Erstarren Wärme an die Umgebung abgeben. Paraffinöl und -wachs wird wegen seiner chemischen Inertheit und seiner Nicht-Toxizität für diesen Zweck besonders bevorzugt.

Andere Kernmaterialien können hautpflegende Materialien wie Hautöl oder Hautcreme sein.

Die Kernmaterialien werden gewöhnlich mit Hilfe eines geeigneten filmbildenden Polymers mikroverkapselt. Bisher wurden Mikrokapseln, die z.B. Paraffinöl oder -wachs als thermostatisierendes Kernmaterial und ein filmbildendes Polymer, wie beispielsweise Acrylharz, als Wandmaterial umfassen, so hergestellt, daß das filmbildende Polymer und das Paraffinöl oder -wachs in einer Düse gleichzeitig versprüht wurden.

Ein Nachteil dieses Herstellungsverfahrens besteht darin, daß die Paraffine bzw. das hautpflegende Material beim Versprühen unvollständig von dem Wandmaterial bedeckt werden, so daß sie teilweise in das Wandmaterial eingelagert sind. Dadurch werden die Kapseln instabil, da das im Wandmaterial enthaltene Paraffinöl oder -wachs, Hautöl oder Hautcreme insbesondere bei höheren Temperaturen aus dem Wandmaterial herauslecken kann oder die Kapsel sogar vollständig zerbrechen kann.

Die Aufgabe der Erfindung ist es, eine Mikrokapsel von eingekapseltem Kernmaterial, insbesondere Paraffinöl und Paraffinwachs bzw. Hautöl oder Hautcreme, zu schaffen, die chemisch und mechanisch stabil ist und so als thermostatisierendes oder hautpflegendes Mittel verwendet werden kann.

Diese Aufgabe wird durch die Mikrokapsel den Anspruchs 1 gelöst.

Die Erfindung stellt Mikrokapseln bereit, welche die Nachteile des Standes der Technik, nämlich das Vorhandensein von Kernmaterial in der äußeren Mikrokapselumhüllung, nicht aufweisen. Der Vorteil der Erfindung liegt darin, daß um eine erste Wandschicht der Mikrokapsel herum, welche gemäß dem oben beschriebenen Verfahren Einlagerungen von Kernmaterial enthalten kann, eine diskret ausgebildete, kein Kernmaterial enthaltende weitere Wandschicht mit einer in sich geschlossenen Oberfläche angeordnet ist. Somit weisen die Mikrokapseln eine stabile äußere Umhüllung auf, wodurch wird ein Klebrigwerden oder Ausschwitzen von Kernmaterial oder ein Zerbrechen aufgrund von Instabilität vermieden wird.

Die Weiterbildung der Erfindung gemäß Anspruch 2 hat Mikrokapseln zum Gegenstand, die als wärmeaufnehmende oder wärmeabgebende Partikel dienen können.

Bevorzugte thermostatisierende Kernmaterialien in der vorliegenden Erfindung sind Paraffine, da sie, wie schon erwähnt, chemisch inert und nicht-toxisch sind. Besonders bevorzugt sind die Paraffine n-Tetradecan (Fp. Schmelzpunkt 5,9 °C), n-Pentadecan (Fp. 10,0 °C), n-Hexadecan (Fp. 18,2 °C), n-Heptadecan (Fp. 22,0 °C), n-Octadecan (Fp. 28,2 °C), n-Nonadecan (Fp. 32,1 °C) und n-Eicosan (Fp. 36,8 °C) (Anspruch 3). Es können aber auch andere organische Kernmaterialien verwendet werden, wie z.B. 2,2-Dimethyl-1,3-propandiol (DMP) oder 2-Hydroxymethyl-2-methyl-1,3propandiol.

Allgemein liegen die Schmelzpunkte der Kernmaterialien der erfindungsgemäßen Mikrokapseln vorzugsweise im Bereich von 5 °C bis 40 °C. Ein bevorzugter Schmelzpunktsbereich der Kernmaterialien liegt zwischen 20 °C und 40 °C (Anspruch 4).

Mikrokapseln, wie sie im Anspruch 5 angegeben sind, können als die Haut pflegende Beschichtung verwendet werden.

Mit der Weiterbildung der Erfindung gemäß Anspruch 6 wird erreicht, daß man die Mikrokapseln in einem frühen Ausrüstungsschritt mit einem Stoff verbinden kann, ohne daß sie in weiteren Ausrüstungschritten beschädigt werden. Im letzten Ausrüstungsschritt oder einem gesonderten Behandlungsschritt, wird dann die äußerste Wandschicht der Mikrokapseln entfernt, so daß ihr Inhalt dann unter Zerstörung der nun freigelegten Rest-Kapselwand freigegeben werde kann, z.B. durch Aufscheuern dieser Kapselwand.

Das Wandmaterial der Mikrokapseln besteht vorzugsweise aus filmbildendem Polymer (Anspruch 7).

Die Materialien für die erste und zweite Wand können unterschiedlich (Anspruch 8) oder auch identisch sein (Anspruch 9).

Bevorzugte Wandmaterialien sind Acrylharze, Polyethylen, Polypropylen, Ethylen-Propylen-Copolymer, Ethylen-Propylen-Dien-Terpolymer, Acrylate und Methacrylate, Polyester, Polystyrol und Silicone (Anspruch 10). Unter diesen Materialien ist Acrylharz für beide Wandschichten speziell bevorzugt, da es besonders stabile Wände liefert, die mechanische Belastungen, wie einer Reibung an der Haut auf der Innenseite von Kleidungsstücken, ohne weiteres standhalten können.

In Anspruch 11 ist ein besonders zweckmäßiges Verfahren zur Herstellung der erfindungsgemäßen Mikrokapseln beschrieben. Die Unteransprüche 12 bis 14 sind bevorzugte Ausgestaltungen des Verfahrens.

Das bevorzugte Verfahren zur Herstellung der erfindungsgemäßen Mikrokapseln umfaßt zwei Stufen:

In einer ersten Stufe werden Paraffinöl oder flüssiges Paraffinwachs oder ein anderes flüssiges oder cremeartiges Kernmaterial zusammen mit einer Lösung eines Wandmaterials durch eine Düse versprüht und ggf. anschließend getrocknet. Dies kann speziell so bewerkstelligt werden, daß durch Herausdrücken des Kernmaterials aus einer Kernbildungsdüse Tropfen geformt werden und weiter aus einer der Kernbildungsdüse benachbarten Wandbildungsdüsen-Anordnung das Wandmaterial so abgegeben wird, daß sich das Wandmaterial um den Kerntropfen legt und eine Wand bildet. Die Wandschicht nach der ersten Stufe ist jedoch nicht vollständig in sich geschlossen, sondern enthält auch Kernmaterial. Im Anschluß können die Mikrokapsel-Zwischenprodukte der Erfindung zur Stabilisierung bei der Lagerung und weiteren Verarbeitung unter den Erstarrungspunkt des Kernmaterials abgekühlt werden (Anspruch 15).

In der zweiten Stufe des Verfahrens werden die in der ersten Stufe gewonnenen Mikrokapsel-Zwischenprodukte im Fließzustand in einem Wirbelbettverfahren bei möglichst tiefer Temperatur (damit die Mikrokapsel-Zwischenprodukte nicht zerstört werden) mit einer Lösung des gleichen oder eines anderen Wandmaterials wie bzw. als in der ersten Stufe besprüht.

Bevorzugt wird eine Vakuum-Wirbelschichtanlage verwendet, in deren Vakuum das Lösungsmittel von der Oberfläche der Mikrokapseln verdampft und einen geschlossenen Film aus Wandmaterial hinterläßt. Derartige Wirbelbettverfahren sind an sich in der Technik bekannt. Beispielsweise eignet sich für die zweite Stufe der vorliegenden Erfindung eine Vakuum-Wirbelschichtanlage mit einem Lösungsmittel-Rückgewinnungssystem, wie sie beispielsweise beim Aufsprühen von Retardfilmen auf Arzneimittelepellets verwendet wird. Eine derartige im Handel erhältliche Vakuum-Wirbelschichtanlage ist beispielsweise die Glatt-R-Vakuum-Wirbelschichtanlage.

Falls die Mikrokapseln am Ende der zweiten Verfahrensstufe noch nicht vollständig frei von Lösungsmittel sind, werden sie in einem gesonderten Trocknungsschritt getrocknet (Anspruch 13).

Die zweite Stufe des erf indungsgemäßen Verfahrens liefert Mikrokapseln, die eine stabile, kein Kernmaterial enthaltende Außenwandschicht aufweisen, thermisch beständig und auch mechanisch sehr stabil sind.

Aus diesem Grund sind die Mikrokapseln auch geeignet, auf die Innenseite von Kleidungsstücken aufgebracht zu werden (Anspruch 16). Die Reibung mit dem Körper reicht nicht aus, um die Mikrokapseln zu zerstören, und so können sie eine thermostatisierende Wirkung am Körper erzielen.

Mit der Weiterbildung der Erfindung gemäß Anspruch 17 wird ein einfaches und sicheres Herstellen der Beschichtung auf der Innenseite eines Kleidungsstücks erzielt.

Dabei kann man gemäß Anspruch 18 ein Kleidungsstück insgesamt so ausrüsten, daß es sowohl thermostatisierend wirkt als auch einen hautpflegenden Effekt gewährleistet.

Mit der Weiterbildung gemäß Anspruch 19 wird erreicht, daß trotz der Beschichtung eine Luftdurchlässigkeit des beschichteten Kleidungsstücks gegeben ist.

Rastergrößen für das Aufdrucken der Beschichtung, wie sie im Anspruch 20 angegeben sind, lassen sich gut realisieren. Trotzdem wirkt die aufgedruckte Beschichtung im wesentlichen auf der Haut homogen.

Jeanshosen sind ein besonders bevorzugtes Kleidungsstück für diese Verwendung der erfindungsgemäßen Mikrokapseln (Anspruch 21).

Gemäß Anspruch 22 kann man ein waschbares Kleidungsstück thermostatisierend oder hautpflegend ausrüsten.

Anspruch 23 gibt bevorzugte Bindemittel an, mit denen Mikrokapseln mit einem Kleidungsstück verbunden werden können. Beispiele sind: Polyurethan, Nitrilgummiarten, Chloropren-Gummiarten, Polyvinylalkohol, Ethylen-Vinylacetat-Copolymere, Acrylharze, Silicone, z.B. Silconelastomere, Stärke und Cellulose. Siliconelastomere sind besonders bevorzugt.

Die thermostatisierende Wirkung wird verstärkt, wenn man ein Gemisch von Mikrokapseln verwendet, wobei jede der Komponenten des Gemischs ein Paraffinöl oder -wachs mit einem speziellen Schmelzpunkt innerhalb des Bereichs von 5 °C bis 40 °C aufweist und die Schmelzpunkte der Paraffin-Komponenten über den ganzen Bereich von 5 °C bis 40 °C verteilt sind (Anspruch 24).

Auftragsmengen der Beschichtung, wie sie im Anspruch 25 angegeben sind, stellen einerseits sicher, daß der gewünschte thermostatisierende und/oder hautpflegende Effekt auch über längere Zeit erhalten wird; andererseits wird durch die Beschichtung der Griff des textilen Materials nicht nennenswert beeinflußt.

Eine Beschichtungsflüssigkeit, die Mikrokapseln der Erfindung, ein Bindemittel und ggf. ein Lösungsmittel und/ oder ein Treibmittel enthält und für die erfindungsgemäße Verwendung benutzt werden kann, wird in Anspruch 26 angegeben. Mit ihrer Hilfe kann ein Benutzer ein Kleidungsstück selbst thermostatisierend und/oder hautpflegend ausrüsten.

Kurze Beschreibung der Zeichnung:
- Figur 1: ist ein schematischer Schnitt durch eine erfindungsgemäße Mikrokapsel;
- Figur 2:: ist ein Schnitt durch ein Kleidungsstück, welches mit einer unteren thermostatisierenden Beschichtung und einer oberen hautpflegenden Beschichtung versehen ist.

Einen Kern 12 einer Mikrokapsel 10 bildet ein Kernmaterial, z.B. Paraffinöl oder -wachs, mit einem Schmelzpunkt zwischen 5 °C und 40 °C. Den Kern 12 umgibt eine erste Wandschicht 14, die gewöhnlich aus einem filmbildenden Polymer gebildet ist, den Kern 12 umhüllt, aber noch eingelagertes Kernmaterial enthalten kann. Die erste Wandschicht 14 ist von einer weiteren Wandschicht 16 umgeben, die kein Kernmaterial enthält und einen abgeschlossenen, kugelschalenähnlichen Aufbau aufweist.

Die Mikrokapsel 10 ist schematisch als Kugel dargestellt, kann aber in der Praxis auch vielerlei andere Formen annehmen, wobei die Durchmesser in einem Bereich von 0,05 *µ*m bis etwa 1000 *µ*m liegen können.

In Abwandlung des oben beschriebenen Ausführungsbeispiels kann man auch Mikrokapseln herstellen, bei denen der Kern 12 durch ein hautpflegendes Material gebildet ist, z.B. ein Hautöl oder eine Hautcreme.

Das hautpflegende Mittel ist im Inneren der beiden Wandschichten 14, 16 dicht und zuverlässig eingeschlossen. Durch mechanisches Zerstören beider Wandschichten 14, 16 wird der Inhalt der Mikrokapsel freigegeben. Ein solches mechanisches Zerstören erfolgt z.B. dann, wenn solche Mikrokapseln in ein Bindemittel eingebettet werden und die so erhaltene Mischung auf ein Kleidungsstück aufgebracht wird, das dann im Reibkontakt mit der Hautoberfläche getragen wird. Dort werden die Mikrokapseln dann nach einander durch das Reiben auf der Haut "angeschliffen", bis ein Zugang zum Kern 12 besteht. Das Kernmaterial wird dann langsam abgegeben und steht für die Pflege der Haut über lange Zeit hinweg zur Verfügung, da das Aufschleifen der verschiedenen Mikrokapseln zu unterschiedlichen Zeitpunkten erfolgt.

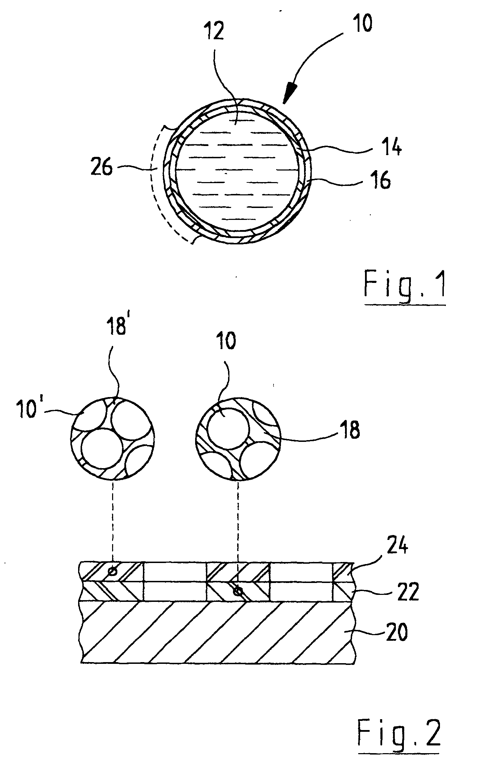

In Figur 2 ist mit 20 ein Kleidungsstück bezeichnet. Dieses trägt eine untere thermostatisierende Beschichtung 22, die im Rastersiebdruck aufgetragen ist. Die Rastergröße wird in der Praxis zwischen 0,5 bis 2 mm gewählt. Über der thermostatisierenden Beschichtung 22 ist registergenau eine hautpflegende Beschichtung 24 vorgesehen. Diese ist ebenfalls im Rastersiebdruck hergestellt.

Die Material-erfüllten Bereiche der beiden Beschichtungen 22, 24 und die nicht mit Material belegten Abschnitte der Beschichtungen 22, 24 fluchten also jeweils, so daß das die beiden Beschichtungen 22, 24 tragende Kleidungsstück 20 insgesamt luftdurchlässig bleibt.

Wie in einer Vergrößerung dargestellt, umfassen die Beschichtungen 22, 24 jeweils ein Bindemittel 18 bzw. 18', in welchem Mikrokapseln 10 bzw. 10' untergebracht sind.

Das Bindemittel ist ein organischer Kunststoff und vorzugsweise aus der nachstehenden Gruppe ausgewählt: Polyurethan, Nitril-Gummiarten, Chloropren-Gummiarten, Polyvinylalkohol, Ethylen-Vinylacetat-Copolymere, Acrylharz, Silicone, z. B. Siliconelastomere, Stärke und Cellulose. Vorzugsweise findet ein Siliconelastomer oder ein vergleichbar abriebfester Kunststoff Verwendung.

Die Beschichtungen 22, 24 haben jeweils ein Flächengewicht von 0,5 bis 200 g/m², vorzugsweise 1 bis 60 g/m².

In Abwandlung des obigen Ausführungsbeispiels kann man auch ein Bindemittel verwenden, welches eine poröse Bindemittel-Schicht ergibt. Ein solches Bindemittel kann dann flächig aufgesprüht oder aufgerakelt werden.

Wird ein Kleidungsstück, wie es in Figur 2 gezeigt ist, getragen, so steht die hautpflegende Beschichtung 24 in ständigem Reibkontakt mit der Hautoberfläche. Das Bindemittel 18' und das Wandmaterial der Mikrokapseln 10' wird durch den Reibkontakt verschlissen, und der Inhalt der Mikrokapseln 10' wird progressiv freigegeben. Hierdurch erhält man einen hautpflegenden Effekt.

Die thermostatisierende Beschichtung wird beim normalen Tragen nicht verändert.

Das Bindemittel 18 ist so gewählt, daß es eine größere Anzahl (etwa 10) Waschvorgänge übersteht.

In Abwandlung des obigen Ausfürhungsbeispieles kann man insbesondere für ein Hautpflegemittel enthaltende Kapseln eine weitere Wand vorsehen, wie sie in Figur 1 bei 26 angedeutet ist.

Die Wand 26 ist aus einem Material hergestellt, welches gegen die Substanzen bzw. Behandlungsflüssigkeiten resistent ist, die in den Ausrüstschritten verwendet werden, denen ein Stoff unterworfen wird, ggf. mit Ausnahme des letzten Ausrüstschrittes. Man kann also diese Kapseln in einem frühen Stadium der Ausrüstung des Stoffes an letzterem anbringen, was im Hinblick auf ein festes Haften vorteilhaft ist. Umgekehrt wird eine gewünscht zerstörbare Wand der Kapseln wieder freigelegt, bevor das Produkt dem Endverbraucher übergeben wird. Dies kann entweder im letzten sowieso vorgesehenen Ausrüstschritt erfolgen, oder in einem zusätzlichen Arbeitsschritt, in dem ein das Material der äußersten Wand angreifendes Mittel zum Einsatz kommt.

## Patentansprüche

1. Mikrokapsel mit einem Kern (12), und mit einer den Kern (12) umgebenen ersten Wandschicht (14), **dadurch gekennzeichnet, daß** die erste Wandschicht (14) von mindestens einer weiteren, kein Kernmaterial enthaltenden Wandschicht (16) umgeben ist.

2. Mikrokapsel nach Anspruch 1, **dadurch gekennzeichnet, daß** das Kernmaterial einen Schmelzpunkt zwischen etwa 5°C und etwa 40°C aufweist.

3. Mikrokapsel nach Anspruch 2, **dadurch gekennzeichnet, daß** der Kern (12) ein Paraffinöl oder ein Paraffinwachs, z.B. n-Tetradecan, n-Pentadecan, n-Hexadecan, n-Heptadecan, n-Octadecan, n-Nonadecan und n-Eicosan, umfaßt.

4. Mikrokapsel nach Anspruch 2 oder 3, **dadurch gekennzeichnet, daß** das Kernmaterial einen Schmelzpunkt zwischen etwa 20 und etwa 40°C aufweist.

5. Mikrokapsel nach Anspruch 1, **dadurch gekennzeichnet, daß** der Kern (12) ein hautpflegendes Mittel, insbesondere ein Hautpflegeöl oder eine Hautcreme umfaßt.

6. Mikrokapsel nach einem der Ansprüche 1 bis 5, **dadurch gekennzeichnet, daß** sie eine äußere Wand (26) aufweist, die gegenüber Substanzen resistent ist, wie sie bei Ausrüstschritten eines Stoffes ggf. mit Ausnahme des letzten Ausrüstschrittes verwendet werden.

7. Mikrokapsel nach einem der Ansprüche 1 bis 6, **dadurch gekennzeichnet, daß** die den Kern (12) umgebende erste Wandschicht (14) und die weitere Wandschicht (16) durch ein filmbildendes Polymer gebildet sind.

8. Mikrokapsel nach Anspruch 7, **dadurch gekennzeichnet, daß** die den Kern umgebende erste Wandschicht (14) und die weitere Wandschicht (16) durch zwei filmbildende Polymere gebildet sind, welche sich in ihren chemischen und/oder physikalischen und/oder mechanischen Eigenschaften unterscheiden.

9. Mikrokapsel nach Anspruch 7, **dadurch gekennzeichnet, daß** die den Kern umgebende erste Wandschicht (14) und die weitere Wandschicht (16) durch das gleiche filmbildende Polymer gebildet sind.

10. Mikrokapsel nach einem der Ansprüche 7 bis 9, **dadurch gekennzeichnet, daß** das filmbildende Polymer aus folgender Gruppe ausgewählt ist: Acrylharze, Polypropylen, Polyethylen, Acrylate und Methacrylate, Polyester, Polystyrol und Silicone.

11. Verfahren zur Herstellung von Mikrokapseln nach einem der Ansprüche 1 bis 10, in dem in einer ersten Verfahrensstufe ein Kernmaterial, das einen Schmelzpunkt zwischen etwa 5°C und 40°C aufweist, in einer Düse mit einem ersten Wandmaterial unter Bildung einer ersten Wandschicht (14) überzogen wird, **dadurch gekennzeichnet, daß** in einer weiteren Verfahrensstufe die in der ersten Verfahrensstufe gewonnenen Mikrokapseln in einem Wirbelbettverfahren mit einem Wandmaterial unter Bildung einer kein Kernmaterial enthaltenden Wandschicht (16) überzogen werden.

12. Verfahren nach Anspruch 11, **dadurch gekennzeichnet, daß** die erste Verfahrensstufe wie folgt durchgeführt wird:
a) Das Kernmaterial wird zu einem Tropfen aus einer Kernbildungsdüse herausgedrückt;
b) aus einer der Kernbildungsdüse benachbarten Wandbildungsdüsenanordnung wird das erste Wandmaterial so abgegeben, daß sich das Wandmaterial um den Kerntropfen legt und eine Wand bildet.

13. Verfahren nach einem der Ansprüche 11 oder 12, **dadurch gekennzeichnet, daß** die in der weiteren Verfahrensstufe erhaltenen Kern- und Wandschichten umfassenden Mikrokapseln vor einer Weiterverwendung getrocknet werden.

14. Verfahren nach einem der Ansprüche 11 bis 13, **dadurch gekennzeichnet, daß** die in der ersten Verfahrensstufe erhaltenen Mikrokapseln vor der weiteren Verfahrensstufe vorgetrocknet werden.

15. Verfahren nach einem der Ansprüche 11 bis 14, **dadurch gekennzeichnet, daß** die in der ersten Verfahrensstufe erhaltenen Mikrokapseln vor der weiteren Verfahrensstufe auf eine unter der Erstarrungstemperatur des Kernmaterials liegende Temperatur abgekühlt werden.

16. Verwendung von Mikrokapseln nach einem der Ansprüche 1 bis 10 als thermostatisierende oder hautpflegende Beschichtung (22; 24) auf der Innenseite eines Kleidungsstücks (20).

17. Verwendung nach Anspruch 16, **dadurch gekennzeichnet, daß** die Mikrokapseln (10) zusammen mit einem Bindemittel (18) auf die Innenseite des Kleidungsstücks (20) aufgebracht, z. B. aufgesprüht, aufgerakelt oder aufgedruckt werden.

18. Verwendung nach Anspruch 16 oder 17, **dadurch gekennzeichnet, daß** auf die Innenseite des Kleidungsstücks (20) zunächst eine thermostatisierende Beschichtung (22) und darüber eine hautpflegende Beschichtung (24) aufgebracht wird.

19. Verwendung nach einem der Ansprüche 16 bis 18, **dadurch gekennzeichnet, daß** die Beschichtung (22; 24) im Rasterdruck aufgebracht wird.

20. Verwendung nach Anspruch 19, **dadurch gekennzeichnet, daß** die Rastergröße, 0,5 bis 2 mm beträgt.

21. Verwendung nach einem der Ansprüche 16 bis 20 **dadurch gekennzeichnet, daß** die Kleidungsstücke Jeanshosen sind.

22. Verwendung nach einem der Ansprüche 16 bis 21 **dadurch gekennzeichnet, daß** ein Bindemittel (18; 18') der Beschichtung (22; 24) nicht wasserlöslich oder in Wasser dispergierbar ist.

23. Verwendung nach einem der Ansprüche 16 bis 22, **dadurch gekennzeichnet, daß** das Bindemittel (18; 18') einen organischen Kunststoff, beispielsweise Polyurethan, Nitril-Gummiarten, Chlororpren-Gummiarten, Polyvinylalkohol, Ethylen-Vinylacetat-Copolymere, Acrylharz, Silicone, z.B Siliconelastomere, Stärke und Cellulose umfaßt.

24. Verwendung nach einem der Ansprüche 16 bis 23, **dadurch gekennzeichnet, daß** eine Mischung von Mikrokapseln (10) verwendet wird, die unterschiedliche Paraffinöle oder -wachse enthalten, die jeweils eines in einer Mikrokapsel eingeschlossen sind.

25. Verwendung nach einem der Ansprüche 16 bis 24, **dadurch gekennzeichnet, daß** die thermostatisierende oder hautpflegende Beschichtung (22; 24) in einer Menge von 0,5 bis 200 g/m², vorzugsweise in einer Menge von 1 bis 60 g/m² aufgebracht wird.

26. Beschichtungsflüssigkeit für eine Verwendung nach einem der Ansprüche 16 bis 25, **dadurch gekennzeichnet, daß** sie wie in den Ansprüchen 1 - 10 definierte Mikrokapseln, ein Bindemittel und ggf. ein Lösungsmittel und/oder ein Treibmittel enthält.
